(19) Europäisches Patentamt
European Patent Office
Office européen des brevets

(11) Publication number: 0 316 809 B1

(12) EUROPEAN PATENT SPECIFICATION

(45) Date of publication of patent specification: 03.11.93

(51) Int. Cl.5: C07C 409/40, C07C 409/42, C07C 407/00, C11D 3/39

(21) Application number: 88118851.0

(22) Date of filing: 11.11.88

# (54) Salts of amino-(poly)percarboxylic acids.

(30) Priority: 13.11.87 IT 2261987

(43) Date of publication of application: 24.05.89 Bulletin 89/21

(45) Publication of the grant of the patent: 03.11.93 Bulletin 93/44

(84) Designated Contracting States: AT BE CH DE ES FR GB IT LI NL SE

(56) References cited: FR-A- 2 239 999

(73) Proprietor: AUSIMONT S.p.A.
Foro Buonaparte, 31
I-20121 Milano(IT)

(72) Inventor: Venturello, Carlo, Dr.
135, via 23 Marzo
I-28100 Novara(IT)
Inventor: Cavallotti, Claudio
3, via Lorenteggio
I-20100 Milano(IT)

(74) Representative: Weinhold, Peter, Dr. et al
Patentanwälte Dipl.-Ing. G. Dannenberg
Dr. P. Weinhold
Dr. D. Gudel
Dipl.-Ing. S. Schubert
Dr. P. Barz
Siegfriedstrasse 8
D-80803 München (DE)

EP 0 316 809 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to new derivatives of organic (poly)peroxyacids, which can be referred to as salts of amino-(poly)percarboxylic acids, and to a process for the preparation thereof as well as their use as bleaching agents.

More particularly, the present invention relates to salts of amino-(poly)percarboxylic acids having the general formula:

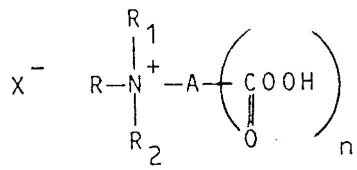

(I)

wherein

R, $R_1$ and $R_2$, which may be the same or different, represent hydrogen or optionally substituted as defined below alkyl or two of said groups R, $R_1$ and $R_2$ are combined to form an optionally substituted as defined below, aliphatic, heterocyclic ring with the nitrogen atom to which they are attached;

A is selected from optionally substituted as defined below alkylene, cycloalkylene, arylene, cycloalkylene-alkylene, alkylene-cycloalkylene, arylene-alkylene or alkylene-arylene, said cycloalkylene or arylene units of the above groups being optionally fused with one or more cycloaliphatic groups and said alkylene units optionally having interspersed $CONR_3$ groups wherein $R_3$ is hydrogen, alkyl or aryl; X represents $HSO_4$ or $CH_3SO_3$; and

n is an integer of from 1 to 6, preferably 1 or 2.

The salts of the above formula (I) are novel compounds and constitute a new class of products that is highly interesting from an industrial point of view.

In fact, they may find general use, similarly to that already known for peroxyacids, in the field of plastics, as polymerization initiator and particularly as oxidizing agents in olefin epoxidation and hydroxylation, and in many other oxidation processes in the field of fine chemicals.

The salts of formula (I), especially those wherein A represents alkylene, are particularly suitable as effective bleaching agents in detergent formulations.

In the past organic peroxyacids have encountered increasing interest in the industrial field, among others due to their excellent properties as bleaching agents in formulations for medium-low temperature washing, and the resulting saving of energy.

Therefore, there is a large number of publications concerned with organic peroxyacid compounds endowed with the required properties of sufficient bleaching activity and, in particular, thermal stability and storage stability or shelf life, the latter characteristics being essential for an industrial application and a widespread use of such compounds. In this context EP-A-83560, 168204 and 206624 may be referred to for merely exemplary purposes.

Therefore many organic straight-chain or cyclic mono- or di-percarboxylic acids are known and used, e.g. in detergent compositions. Examples of already described percarboxylic acids are diperdodecanedioic acid, monoperphthalic acid, diperazelaic acid, substituted diperglutaric and adipic acid.

FR-A-2239 999 discloses the sulfate (not bisulfate) of p-(trimethylammonium)-peroxybenzoic acid. Said salt is the only salt compound mentioned in said document, all of the remaining compounds reported to display a bleaching activity (particularly for teeth) being neutral mono- and di-percarboxylic acids.

According to the present peroxycarboxylation process the oxydation of the substrate (an organic acid or ester or a quaternary salt thereof) is carried out with a concentrated solution of hydrogen peroxide, in concentrated $H_2SO_4$ or $CH_3SO_3H$.

Therefore, the strong acidity of the reaction medium and the presence of a salified or salifiable nitrogen atom of basic character in the starting substrate render said substrate highly soluble in the acidic medium.

Such high solubility makes it impossible to use any of the conventional processes for the isolation of the percarboxylic acid derivative which is formed in the oxidation reaction with hydrogen peroxide. In particular, the generally employed methods of precipitation from the reaction mixture by dilution with large amounts of water, or extraction with an organic solvent selective to the percarboxylic acid product and immiscible with the remainder of the reaction mixture, are not feasible.

Surprisingly, it has been found that the compounds of formula (I), salified on the nitrogen atom with the $HSO_4^-$ or $CH_3SO_3^-$ anion, can be isolated, by means of a novel process which is also a part of the present

invention.

Therefore, one object of the present invention is to provide novel salts of amino-(poly)percarboxylic acids having the above general formula (I).

Another object of the present invention is to provide a simple and inexpensive process for the preparation of said salts of formula (I), in a per se stable form.

A still further object of the present invention is the use of the salts of formula (I) as bleaching agents in detergent formulations, and especially those intended for low-medium temperature use.

These, and still other objects, will become even clearer for those skilled in the art from the following detailed disclosure.

According to the present invention, the salts having the above formula (I), are prepared by a process which is characterized in that a substrate, i.e an amino-(poly)carboxylic acid or ester thereof corresponding to the desired salt of formula (I) or a quaternary salt of said acid or ester, is reacted with (concentrated) $H_2O_2$ in a medium selected from concentrated $H_2SO_4$ and $CH_3SO_3H$ and that the resulting salt of formula (I) is then separated from the reaction mixture by addition of tetrahydrofuran, ethylacetate or a mixture thereof.

Due to their insolubilization in the reaction medium by addition of THF and/or ethylacetate, the percarboxylic acids are thus obtained, generally as stable solids, salified on their nitrogen atom with the $HSO_4^-$ or $CH_3SO_3^-$ anion which optionally is already present on the substrate which is used as the starting material or derives from the reaction medium.

In more detail, the process according to the present invention consists in the peroxycarboxylation reaction of a substrate consisting of the (poly)carboxylic acid, optionally already quaternized on the N-atom (or an ester thereof), in an acid medium composed of concentrated $H_2SO_4$ or $CH_3SO_3H$, with $H_2O_2$ and in the subsequent addition, at the end of the reaction, of a suitable organic solvent which does not dissolve the desired product but is completely miscible with the acid reaction medium (concentrated $H_2SO_4$ or $CH_3SO_3H$) and the excess of $H_2O_2$ and the reaction water. This results in the separation, by insolubilization, of the product of formula (I).

As mentioned above, the substrate which is used as the starting material, corresponding to the compound of formula (I), may be an amino-(poly)carboxylic acid which optionally is already quaternized on the nitrogen atom.

The compounds used as starting materials in the present process are known compounds and/or can be prepared according to conventional techniques.

Nevertheless, when in the substrate at least one of the groups R, $R_1$ and $R_2$ is hydrogen, it has been found to be advantageous from the operational point of view, to carry, out the above preparation process (in the form of $HSO_4^-$ or of $CH_3SO_3^-$ ) by working in the absence of $H_2O_2$, under the same conditions as described above for the peroxycarboxylation reaction, and by separating the obtained quaternary salt which is then peroxidized. When at least one of R, $R_1$ and $R_2$ is hydrogen, it has proved to be advantageous in some cases to use the previously quaternized starting substrate in the peroxidation reaction.

The obtained product is then filtered, washed with solvent, dried, etc., according to the known techniques.

With reference to formula (I) R, $R_1$ and $R_2$ may be identical or different groups. They may represent hydrogen or linear or branched alkyl groups, preferably containing 1 to 10, and particularly 1 to 5, carbon atoms Examples of such groups are methyl, ethyl, n-propyl, i-propyl, n-butyl, i-butyl, sec.-butyl, tert.-butyl, pentyl, i-pentyl, hexyl, octyl and decyl. Moreover, two of the groups R, $R_1$ and $R_2$ may be combined to form a heterocyclic ring together with the nitrogen atom to which they are attached. The resulting heterocyclic ring preferably has 4 to 6 carbon atoms. Particularly preferred are pyrrolidine and piperidine rings.

The above alkyl groups and heterocyclic rings may optionally be substituted by one or more, preferably 1 to 3, substituents. If several substituents are present they may be the same or different. Said substitutents should be inert under the reaction conditions of the peroxydation and also inert towards the resulting percarboxylic acid. Thus said substituents are selected from halogen atoms, i.e F, Cl, Br and I, particularly F and Cl, OH, $NO_2$, lower alkoxy groups, i.e. alkoxy groups having 1 to 6, preferably 1 to 4, carbon atoms, such as, e.g. methoxy, ethoxy, propoxy, i-propoxy and butoxy and carboxylic groups (e.g. $COOR_4$, $OCOR_4$ and $COR_4$ wherein $R_4$ is preferably hydrogen or $C_{1-10}$ alkyl).

A may represent a linear or branched alkylene group $(CH_2)_m$ wherein m preferably is an integer of from 1 to 20, particularly from 1 to 15, such as the alkylene groups derived from the above mentioned alkyl groups (methylene, ethylene, propylene, butylene, hexylene, octylene, decylene, undecylene, duodecylene, tridecylene, pentadecylene, heptadecylene, nonadecylene etc.). A may also represent a (preferably $C_{3-12}$)-cycloalkylene group (e.g cyclopentylene, cyclohexylene, cyclooctylene), a (preferably $C_{6-14}$-)arylene group

(e.g. phenylene, naphthylene, biphenylene, tolylene, xylylene), a (preferably $C_{3-12}$-)cycloalkylene-(preferably $C_{1-5}$-)alkylene group, a (preferably $C_{1-5}$-)alkylene-(preferably $C_{3-12}$-)cycloalkylene group, a (preferably $C_{1-5}$-)alkylene-(preferably $C_{6-14}$-)arylene group or a (preferably $C_{6-14}$-)arylene-(preferably $C_{1-5}$-) alkylene group. Examples of the alkylene, cycloalkylene and arylene units contained in the last mentioned groups have been given above. When there is a cycloalkylene or arylene present in the group A the same may be fused with at least one (preferably $C_{3-12}$-, particularly $C_{3-8}$-)cycloaliphatic group, e.g. cyclopentylene or cyclohexylene.

The group A may carry one or several substituents, preferably 1 to 3. Said substituents are the same as those already given in connection with the groups R, $R_1$ and $R_2$. When the group A comprises a linear or branched alkylene group said group may optionally have interspersed $CONR_3$ units (preferably 1 to 3 such units), wherein $R_3$ is hydrogen, (preferably $C_{1-5}$-)alkyl (e.g. the alkyl groups mentioned above) or aryl (preferably having 6 to 14 carbon atoms, e.g. phenyl).

n in formula (I) is an integer of from 1 to 6, preferably 1 to 3. Particularly preferred are compounds wherein n is 1 or 2.

Esters optionally used as starting materials for obtaining the compounds of formula (I) are preferably the $C_{1-12}$ alkyl esters, e.g the methyl, ethyl, propyl or butyl esters.

Preferred starting substrates for obtaining the corresponding salts of formula (I) are e.g. 4-amino-butyric acid, 3-amino-propionic acid, (carboxymethyl)-trimethylammonium hydroxide or chloride (or monohydrated betaine), 3-piperidine-propionic acid, 11-amino-undecanoic acid, 12-amino-dodecanoic acid, glycyl-glycine, 3-amino-benzoic acid, 5-amino isophthalic acid, 4-amino-phenylacetic acid, 5-amino-valeric acid, 6-amino-caproic acid, L-asparatic acid, N,N-dimethylamino-lauric acid, N,N-dimethylamino-undecanoic acid.

According to a preferred procedure, the peroxycarboxylation of the amino-derivative used as the starting substrate, or its quaternary salt, is carried out by gradually adding $H_2O_2$ having a concentration of from about 70% to about 90% by weight to a solution of the substrate in concentrated $H_2SO_4$,or in $CH_3SO_3H$ and by maintaining the reaction temperature throughout the reaction within the range of from about 15 to about 50 °C, depending on th reactivity of the substrate.

The amount of $H_2SO_4$ or of $CH_3SO_3H$, determined at a concentration of 100%, is not less than 2 moles, e.g. from about 2 to about 30 moles per mole of substrate and is preferably from about 7 to about 10 moles per mole of substrate.

The hydrogen peroxide is used in an amount which is in excess with respect to the substrate, and is not less than 1 mole, e.g. from about 1 to about 6 moles per mole of substrate, and preferably from about 1.2 to about 2 moles per mole of substrate.

The reaction time depends on the nature of the substrate, on the reaction temperature, and on the total $H_2SO_4/H_2O$or $CH_3SO_3H/H_2O$ molar ratio at the end of the reaction. Said ratio is preferably kept at not less than 1.5, e.g. from about 1.5 to about 10, and preferably from 4 to 6, by adjusting the various relative parameters.

Reaction times between approximately 30 minutes and 4 hours have been shown to be operative, and generally a reaction time of from approximately 1 hour to approximately 2 hours is sufficient.

The amount of tetrahydrofuran and/or ethyl acetate used is preferably not lower than 4 liters/mole substrate, and furthermore it is preferred to add it at a temperature not higher than approximately 10 °C.

The final reaction mixture, before separation of the salts of formula (I), may optionally be subjected to a phlegmatization process.

The obtained salts of formula (I) are usually solid at room temperature. They may be especially useful in formulations of detergent compositions, e.g. granular formulations, as bleaching agents in solution over a wide temperature range.

The detergent compositions may be formulated according to the conventional techniques in this field together with other components and/or additives.

The present invention will now be illustrated in still further detail by the following examples, which are not to be construed as limiting the scope of the present invention.

The products prepared in the examples were characterized by elemental analysis, by determining their content of active oxygen (by iodometric titration), and by using Fourier Transform Infrared Spectroscopy (FI-IR).

Examples 5, 6 and 12 were carried out with separately salified substrates.

## Example 1

A beaker, equipped with stirrer, thermometer and external bath was charged with 30 g (0.312 mole) of methanesulfonic acid.

The internal temperature was raised to 35 °C to 40 °C and 4 g (0.0387 mole) of 4-amino-butyric acid were added under stirring for 15 minutes.

The above temperature was maintained until the amino-carboxylic acid was completely dissolved and then the temperature was lowered to 15 °C and 3.2 g of $H_2O_2$ (85%, 0.08 mole) were gradually added under stirring so that the temperature was maintained below 25 °C. The stirring was continued for 1.5 hours at 20 - 25 °C.

At the end, the reaction mixture was poured into 300 ml of tetrahydrofuran (THF) maintained under stirring at 0 - 10 °C. After 1 hour of stirring, the separated product was filtered over a porous septum, washed with THF (2 x 30 ml), then with diethyl ether (2 x 30 ml), and was finally kept in a desiccator filled with $CaCl_2$ under vacuum at room temperature for 1 hour.

7.5 g substantially pure crystalline (3-percarboxypropyl)-ammonium methanesulfonate were obtained. Yield: 89.8%.

Elemental Analysis:

Calculated for $C_5H_{13}O_6NS$: C: 27.90%; H: 6.08%; N: 6.50%; O (active): 7.43%; $CH_3SO_3H$: 44,65%.
Found: C: 27.99%; H: 6.00%; N: 6.49%; 0 (active): 7.42%; $CH_3SO_3H$: 43.13%.
Melting Point: 44 °C (with decomposition).

By following the same procedure, (4-percarboxybutyl)-ammonium-and (5-percarboxypentyl)-ammonium methane sulfonate were prepared by starting from the corresponding substrates.

4-percarboxybutyl derivative: calculated 0 (active): 6.98%; found O (active): 6.80%.
5-percarboxypentyl derivative: calculated and found O (active): 6.57%.

Example 2

2 g (0.0224 mole) of 3-amino-propionic acid were completely dissolved in 15 g of $H_2SO_4$ (96%; about 0.146 mole) and, by following the procedure of Example 1, then treated with 1.8 g of $H_2O_2$ (85%; 0.045 mole), thereafter continuing stirring for 1 hour. At the end, always following the procedure of Example 1, the reaction mixture was poured into 200 ml of THF. By proceeding as described in Example 1,4 g of crystalline, substantially pure (2-percarboxyethyl)-ammonium bisulfate were obtained. Yield: 87.8 %.

Elemental Analysis:

Calculated for $C_3H_9O_7NS$: C: 17.73%; H: 4.46%; N: 6.89%; 0 (active) : 7.87%; $H_2SO_4$ : 48.27%.
Found: C: 18.02%; H: 4.79%; N: 6.91%; 0 (active): 7.86%; $H_2SO_4$:47.98%.

Example 3

By following the procedure of Example 1, 3 g (0.0222mole) of trimethyl ammonium (carboxymethyl) hydroxide (betaine mono-hydrate) were completely dissolved in 30 g of $H_2SO_4$ (96%; 0.292 mole) and then treated with 3 g of $H_2O_2$ (85%; 0.075 mole), so as to maintain a temperature of not higher than 30 °C. Thereafter stirring was continued at 30 °C for 4 hours. At the end, by following the procedure of Example 1, the reaction mixture was poured into 400 ml of THF. The work-up was carried out as described in Example 1. 4.2 g of crystalline trimethyl-(percarboxymethyl)ammonium bisulfate were obtained, having a purity of 90% (active oxygen content: 6.22 %; theoretical value 6.92%); corresponding to a yield of 73.7%.

Elemental Analysis:

Calculated for $C_5H_{13}O_7NS$: C: 25.97%; H: 5.66%; N: 6.06%; 0 (active): 6.92%; $H_2SO_4$ : 45.42%.
Found: C: 25.96%; H: 5.72%; N: 6.1%; 0 (active): 6.22%; $H_2SO_4$:45.89%.
Melting Point: 52 °6 (with decomposition).

Example 4

By following the procedure of Example 1, 4.7 g (0.0287 mole) of 3-piperidine propionic acid were dissolved in 36 g (0.374 mole) of methanesulfonic acid and then treated with 6 g of $H_2O_2$ (70%; 0.123 mole), so as to maintain a temperature of not higher than 20 °C. Thereafter stirring was continued at 15 - 20 °C for 1 hour. At the end, always following the procedure of Example 1, the reaction mixture was poured

into 400 ml of THF. The work-up was then carried out as described above. 7.2 g of crystalline substantially pure methane sulfonic acid salt of 3-piperidine-perpropionic acid were obtained.
Yield: 93%.

Elemental Analysis:

Calculated for $C_9H_{19}O_6NS$: C: 40.13%; H: 7.11%; N: 5.20%; 0 (active): 5.94%; $CH_3SO_3H$: 35.68%.
Found: C: 39.73%; H: 6.94%; N: 5.08%; 0 (active); 5.93%; $CH_3SO_3H$: 35.9%.
Melting Point: 132 °C (with decomposition).

Example 5

5 g (0.0248 mole) of 11-amino-undecanoic acid were added, slowly and under stirring, to 15 g of methanesulfonic acid in a 50 ml beaker, maintaining the temperature at or below 40 °C by using a cooling bath; stirring was then continued at 35 - 40 °C to complete dissolution.

The mixture was then poured into 150 ml of ethyl acetate maintained under stirring at 10 °C. Stirring was continued for 30 minutes. The precipitated (10-carboxydecyl)-ammonium methane-sulfonate was filtered over a porous septum, washed first with ethyl acetate (2 x 30 ml) and then with $Et_2O$ (2 x 30 ml), whereafter it was dried under vacuum at room temperature over $CaCl_2$. 7 g of product were obtained.

6.6 g (0.0222 mole) of (10-carboxydecyl)-ammonium methanesulfonate were dissolved in 13 g of $CH_3SO_3H$ at room temperature. 1.1 g of $H_2O_2$ (85%; 0.0275 mole) were added slowly and under stirring, so as to maintain the temperature at or below 15 °C. Thereafter stirring was continued for 30 min at 10 - 15 °C.

At the end, by following the procedure of Example 1, the reaction mixture was poured into 300 ml of ethyl acetate. The subsequent work-up was carried out as described in Example 1. 4.9 g of crystalline, substantially pure (10-percarboxydecyl)-ammonium methanesulfonate were obtained. Yield: 70.4%.

Elemental Analysis:

Calculated for $C_{12}H_{27}O_6$ NS: C: 45.98%; H: 8.68%; N: 4.46%; 0 (active): 5.10%; $CH_3SO_3H$: 30.66%.
Found: C: 45.83%; H: 8.78%; N: 4.48%; 0 (active): 5.09%; $CH_3SO_3H$: 30.79%.
Melting Point: 63 °C (with decomposition).

Example 6

5g (0.0232 mole) of 12-amino-dodecanoic acid were treated, according to the procedure of Example 5, with 25 g of $CH_3SO_3H$.

In the above procedure tetrahydrofuran was used instead of ethyl acetate. 6.7 g of (11-carboxyundecyl)-ammonium methane-sulfonate were obtained.

8.3 g (0.0267 mole) of the above salt were dissolved in 16 g of $CH_3SO_3H$ at ambient temperature.

1.3 g of $H_2O_2$ (85%; 0.0325 mole) were added slowly and under stirring by following the procedure of Example 5. At the end, the procedure of Example 1 was followed and 8 g of crystalline and substantially pure (11-percarboxyundecyl)-ammonium methanesulfonate were obtained. Yield: 91.4%.

Elemental Analysis:

Calculated for $C_{13}H_{29}O_6NS$: C: 47.68%; H: 8.93%; N: 4.27%; 0 (active): 4.88%; $CH_3SO_3H$: 29.35%.
Found: C: 47.08%; H: 8.82%; N: 4.25%; 0 (active): 4.87%; $CH_3SO_3H$: 29.21%.
Melting Point: 92 °C (with decomposition).

Example 7

By following the procedure of Example 1, 5 g (0.0378 moles) of glycyl-glycine were completely dissolved in 25 g of $CH_3SO_3H$and then treated with 3.6 g of $H_2O_2$ (85%). Thereafter stirring was continued at 20 - 25 °C for 2 hours. Following the procedure of Example 1 and using 400 ml of THF, 8.8 g of crystalline methane sulfonic acid salt of glycyl-amino-peracetic acid were isolated, having a purity of 91.5 % (active oxygen content: 5.99 %; theoretical value: 6.55), corresponding to a yield of 87.2%.

Elemental Analysis:

Calculated for $C_5H_{12}O_5N_2S$: C: 24.59%; H: 4.96%; N: 11.47%; 0 (active): 6.55%; $CH_3SO_3H$, 39.35%.
Found: C: 24.58%; H: 5.32%; N: 11.51%; 0 (active) 5.99%; $CH_3SO_3H$: 39.20%.
Melting Point: 97°C (with decomposition).

Example 8

The procedure of Example 1 was repeated, substituting 4-amino-butyric acid with 3-amino-benzoic acid (4 g; 0.0291 mole) and using 2.9 g of $H_2O_2$ (85%; 0.0725 mole) instead of 3.2 g. 6.6 g of crystalline methane sulfonic acid salt of 3-amino-perbenzoic acid were obtained having a purity of 98%.
Yield: 89.8%.

Elemental Analysis

Calculated for $C_8H_{11}O_6NS$: C: 38.55%; H: 4.45%; N: 5.62%; O (active): 6.42%; $CH_3SO_3H$: 38.55 %.
Found: C: 39.0%; H: 4.22%; N: 5.62%; 0 (active): 6.29%; $CH_3SO_3H$: 37.9 %.
The product decomposed at 114°C without melting.

Example 9

The procedure of Example 8 was repeated, substituting 3-amino-benzoic acid with 5-amino-isophthalic acid (2 g; 0.011 mole) and using 32 g of $CH_3SO_3H$ instead of 30 g, 2.7 g of $H_2O_2$ (85%; 0.0675 mole) instead of 2.9 g and ethyl acetate (800 ml) instead of THF.

3.1 g of crystalline and substantially pure methane sulfonic acid salt of 5-amino-di-per-isophthalic acid were obtained.
Yield: 91%.

Elemental Analysis:

Calculated for $C_9H_{11}O_9NS$: C: 34.95%; H: 3.58%; N: 4.53%; 0 (active): 10.34%; $CH_3SO_3H$: 31.07%.
Found: C: 34.96%; H: 3.85%; N: 4.30%; 0 (active): 10.33%; $CH_3SO_3H$: 30.96%.
The product decomposed at 122°C without melting.

Example 10

The procedure of Example 8 was repeated, substituting 3-amino-benzoic acid with 4-amino-phenylacetic acid (3g; 0.0198 mole) and using 19g of $CH_3SO_3H$ instead of 30 g, 2.2 g of $H_2O_2$ (85%; 0.055 mole) instead of 2.9 g and ethyl acetate (300 ml) instead of THF.
5.2 g of crystalline and substantially pure methane sulfonic acid salt of 4-amino-phenyl-peracetic acid were obtained.
Yield: 98.5 %.

Elemental Analysis

Calculated for $C_9H_{13}O_6NS$: C: 41.06%; H: 4.98%; N: 5.32%; 0 (active): 6.07%; $CH_3SO_3H$: 36.5%.
Found: C: 40.71%; H: 5.24%; N: 4.95%, 0 (active): 6.00%; $CH_3SO_3H$: 35.82%.
The product decomposed at 134°C without melting.

Example 11

The procedure of Example 2 was repeated by substituting sulfuric acid with methanesulfonic acid (15 g; 0.156 mole).

4.4 g of crystalline and substantially pure methane sulfonic acid salt of 3-amino-perpropionic acid were obtained. Yield: 98%.

Elemental Analysis

Calculated for $C_4H_{11}O_6NS$: C: 23.87%; H: 5.51%; N: 6.96%; 0 (active): 7.95%; $CH_3SO_3H$: 47.76%.
Found: C: 23.88%; H: 5.66%; N: 6.70%; 0 (active): 7.95%; $CH_3SO_3H$: 48.02%.

Example 12

5 g (0.056 mole) of 3-amino-propionic acid were added, slowly and under stirring, to 15 g of sulfuric acid in a 50 ml beaker, maintaining the temperature at or below 40°C by using a cooling bath. The stirring was then continued at 35 - 40°C to complete dissolution.

The mixture was then poured into 150 ml of tetrahydrofuran, maintained under stirring at 10°C. The stirring was continued for 30 min.

The separated bisulfate of 3-amino-propionic acid was filtered over a porous septum, washed first with tetrahydrofuran (2 x 30 ml) and then with $Et_2O$ (2 x 30 ml) and then dried under vacuum at room temperature over $CaCl_2$.
The procedure of Example 2 was repeated, substituting 3-amino-propionic acid with the above obtained bisulfate (4.18 g, 0.0224 mole). Analogous results were obtained.

Example 13

The procedure of Example 3 was repeated, substituting (carboxy-methyl) trimethyl ammonium hydroxide with the corresponding chloride (3.41 g; 0.0222 mole). Analogous results were obtained.

Example 14

5 g (0.0248 mole) of 11-amino-undecanoic acid were converted into the corresponding bisulfate by working as described in Example 5, but substituting methanesulfonic acid with sulfuric acid. 6.5 g of product were obtained.

6 g (0.0201 mole) of the above obtained bisulfate were dissolved in 9 g of $H_2SO_4$ (96%; 0.0882 mole) at room temperature.

2.1 g of $H_2O_2$ (85%; 0.0525 mole) were then added slowly and under stirring, so as to maintain the temperature at or below 15°C. Thereafter stirring was continued for 30 minutes at 10 - 15°C. At the end, by working according to the procedure of Example 1, the reaction mixture was poured into 300 ml of ethyl acetate, proceeding as above described.

6 g of crystalline and substantially pure bisulfate of 11-amino-perundecanoic acid were obtained.
Yield: 95%.
The composition was confirmed by the elemental analysis. 0 (active): calculated 5.07%; found 5.04%.

By working according to an analogous procedure, the bisulfate of 12-amino-perdodecanoic acid was prepared.
0 (active): calculated 4.85%; found 4.85%.

Example 15

A beaker equipped with stirrer, thermometer and outer bath was charged with 15 g of $H_2SO_4$ (96%; 0.147 mole). The internal temperature was kept at 0°C and, under agitation and during 10 minutes, 4.8 g of $H_2O_2$ (85%; 0.12 mole) were added by maintaining the isotherm at a temperature lower than +5°C. Then 6 g of L-aspartic acid (0.045 mole) were added, maintaining the temperature at 15°C. Stirring was continued at 15°C for 1 hour.

At the end, the reaction mixture was poured into 250 ml of ethyl acetate maintained under stirring at 0°C. After 30 minutes of stirring at this temperature, the separated crystalline product was filtered over a porous septum, under vacuum and washed with 2 x 30 ml of ethylacetate and then with 2 x 30 ml of diethyl ether. The product was then dried for 1 hour, under vacuum, at room temperature in a $CaCl_2$ drier.

8.5 g of crystalline and substantially pure bisulfate of 2-amino-mono-persuccinic acid were obtained.
Yield: 76%.

Elemental Analysis

Calculated for $C_4H_9O_9NS$: C: 19.43%; H: 3.67%; N: 5.66%; 0 (active): 6.47%; $H_2SO_4$: 39.67%.
Found: C: 19.43%; H: 3.88%; N: 5.63%; 0 (active): 6.46%; $H_2SO_4$: 39.1.
Melting Point: 98 °C (with decomposition).

Example 16

By working according to the procedure of Example 1, 2 g of $H_2O_2$ (85%; 0.05 mole) were added, under stirring, to 6.3 g of $H_2SO_4$ (96%; 0.0617 mole) so as to maintain the temperature at $\leqq$ 5 °C. 2 g of 4-amino-butyric acid (0.0194 mole) were slowly added to maintain the temperature below +10 °C. The stirring was continued for 30 minutes at 10 °C. At the end, by working according to the procedure of Example 1, the reaction mixture was poured into 60 ml of ethyl acetate, maintained under stirring at 0 °C, by proceeding as described above.
3.8 g of crystalline, substantially pure bisulfate of 4-amino-perbutyric acid were obtained.
Yield: 90%.

Elemental Analysis

Calculated for $C_4H_{11}NSO_7$: C: 22.12%; H: 5.1%; N: 6.45%; 0 (active): 7.36%; $H_2SO_4$: 45.15%.
Found: C: 22.22%; H: 5.32%; N: 6.39%; 0 (active): 7.35%; $H_2SO^4$: 44.93%.
Melting Point: 40 °C (with decomposition).

Example 17

By working according to the procedure of Example 1, 3 g (0.0229 mole) of 6-amino-caproic acid were added, under stirring at 0 - 5 °C, to a mixture of 7.5 g of $H_2SO_4$ (96%; 0.0734 mole) and 2.4 g of $H_2O_2$ - (85%; 0.06 mole), so as to maintain the temperature at or below 10 °C.
Stirring was continued for 30 minutes at 10 °C. The reaction mixture was poured into 100 ml of ethyl acetate, by proceeding as in the above Example 1.
4 g of crystalline, substantially pure bisulfate of 6-amino-percaproic acid were obtained. Yield: 71%.

Elemental Analysis

Calculated for $C_6H_{15}NSO_7$: C: 29.38%; H: 6.16%; N: 5.71%; 0 (active): 6.52%; $H_2SO_4$: 39.99%.
Found: C: 29.97%; H: 6.39%; N: 5.69%; 0 (active): 6.51%; $H_2SO_4$:39.01%.
Melting Point: 47 °C (with decomposition).

Example 18

By working according to the procedure of Example 1, 2 g of 5-amino-valeric acid (0.0171 mole) were added to a mixture of 4.9 g of $H_2SO_4$ (96%; 0.048 mole) and 1.6 g of $H_2O_2$ (85%; 0.04 mole), maintained under stirring at 0 - 5 °C, so as to maintain the temperature at or below 10 °C. Stirring was continued for 30 minutes at 10 °C. The reaction mixture was poured into 100 ml of ethyl acetate, by proceeding according to the procedure of Example 1. 3.4 g of substantially pure bisulfate of 5-amino-pervaleric acid were obtained.
Yield: 86%.

Elemental Analysis

Calculated for $C_5H_{13}NSO_7$: C: 25.97%; H: 5.23%; N: 6.05%; 0 (active): 6.92%; $H_2SO_4$: 42.41%.
Found: C: 25.6%; H: 5.8%; N: 5.93%; 0 (active): 6.91%; $H_2SO_4$:41.88 %.
Melting Point: 64 °C (with decomposition).

Example 19

3 g (0.0088 mole) of methane sulfonate of N,N-dimethylaminolauric acid were completely dissolved in 4.5 g of $CH_3SO_3H$ (0.0468 mole) at 15 °C and then treated with 0.5 g (0.0125 mole) of $H_2O_2$ (85%), so as to maintain the temperature at or below 15 °C. The stirring was continued for 45 minutes at 15 °C.

The reaction mixture was then poured into 70 ml of tetrahydrofuran maintained under stirring at -10 °C. By proceeding according to the procedure of Example 1, 1.5 g of crystalline, substantially pure methane sulfonate of N,N-dimethyl-amino-perlauric acid were obtained.
Yield: 48%.

Elemental Analysis

Calculated for $C_{15}H_{33}O_6NS$: C: 50.68%; H: 9.35%; N: 3.94%; 0 (active): 4.5%; $CH_3SO_3H$: 27.03%.
Found: C: 50.28%; H: 9.35%; N: 3.91%; 0 (active): 4.49%; $CH_3SO_3H$: 26.91%.
Melting Point: 75 °C (with decomposition).

Example 20

2 g (0.0088 mole) of N,N-dimethyl-amino-undecanoic acid were completely dissolved in 2.9 g of $H_2SO_4$ (96%; 0.0284 mole).

0.9 g of $H_2O_2$ (85%; 0.0226 mole) were then added to the mixture under stirring at 0 - 5 °C, so as to maintain the temperature at or below 10 °C.

Stirring was continued for 1 hour at 15 °C. The reaction mixture was poured into 80 ml of ethyl acetate, maintained under stirring at -30 °C. By working according to the procedure of Example 1, 2.2 g of crystalline, substantially pure bisulfate of N,N-dimethyl-amino-perundecanoic acid were obtained.
Yield: 73%.

Elemental Analysis

Calculated for $C_{13}H_{29}NSO_7$: C: 45.46%; H: 8.51%; N: 4.08%; 0 (active): 4.66%; $H_2SO_4$: 28.55%.
Found: C: 45.31%; H: 8.55%; N: 4.02%; 0 (active): 4.65%; $H_2SO_4$:28.47%.
Melting Point: 46 °C (with decomposition).

Example 21 (Application Example)

Bleaching tests were carried out with the novel salts listed in the annexed Tables 1 and 2, at alkaline pH (Table 1) and acid pH (Table 2). The following substances served as comparison:

(a) H 48 (Mg salt of monoperphthalic acid), a commercial per-acid known in the detergent art, and manufactured by INTEROX Chemical Ltd., London, U.K. (Tables 1 and 2).

(b) Perborate + perborate activator system, which, as known, forms a peracid (peracetic acid) in situ when both products are dissolved in water and which represents the presently most widely used form for the purpose of obtaining a bleaching action at medium-low temperatures (≦60 °C) and wherein, as the activator, TAED (tetraacetylethylenediamine) was selected, in a stoichiometric amount with respect to the amount of the perborate (Table 1).

(c) Sodium perborate ($NaBO_3.4H_2O$) alone (PBS) (Table 1).

All tests were carried out at constant temperature (60 °C), with an initial concentration of total active oxygen in the bleaching solution (identical for all products) of equal to 200 mg/l.

Process

For each test, 500 ml of deionized water, contained in a 1000 ml flask equipped with a condenser, was heated to a temperature of 60 °C and adjusted to a pH of 9.5 (by adding a few drops of an NaOH solution) (Table 1) and of 2 - 3 (by adding a few drops of diluted $H_2SO_4$) (Table 2). Then the bleaching product was added with stirring, in amounts shown in the following Tables, and immediately thereafter, two cotton specimens of 10 cm x 10 cm stained with standard stains of red wine at EMPA INSTITUTE of St. Gallen (Switzerland), and marked with the "EMPA 114" mark, were added.

The system subsequently was kept under stirring for 60 minutes and, at the end of this time, the specimens, rinsed under running water, were dried and ironed, and were then subjected to an evaluation of the bleaching effect by means of a measurement of the whiteness degree by reflectometry. The results are reported in the following Tables 1 and 2, wherein the data are expressed as % Bleaching, defined as:

$$\% \text{ Bleaching} = \frac{A - B}{C - B} \times 100\%$$

wherein:

A = degree of whiteness (%) of the specimen bleached after the test;
B = degree of whiteness (%) of the speciment before the test;
C = degree of whiteness (%) of the completely bleached specimen.

The degree of whiteness was measured by means of an Elrepho Zeiss reflectometer, assuming MgO = 100% of whiteness, and using a filter N.6. (λ = 464 nm).

The data listed in Table 1, concerning tests carried out at alkaline pH, show that the peracid salts of the present invention have a bleaching power which may be compared to the bleaching power of H 48 and in some cases is even higher than that.

Likewise, the results, expressed as % bleaching, listed in Table 2, show that the tested products have a bleaching power in acid solution which is particularly high and much higher than that of H 48.

This is particularly surprising in consideration of the fact that the peroxidic compounds generally show a very modest and sometimes negligible bleaching activity at acid pH.

**TABLE 1**

**Test carried out at alkaline pH (9.5)**

| Compound | Amounts used in the tests (grams) | Initial concentration of total active oxygen (mg/l) | % Bleaching |
|---|---|---|---|
| -Example 4 (titer = 5.93% of active oxygen) | 1.69 | 200 | 76.7 |
| - Example 7 (titer = 5.99% of active oxygen) | 1.67 | 200 | 74.3 |
| -Example 6 (titer = 4.87% of active oxygen) | 2.05 | 200 | 44.9 |

TABLE 1 (continued)

| | | | |
|---|---|---|---|
| -Example 3 (titer = 6.22% of active oxygen) | 1.61 | 200 | 72.8 |
| -Example 11 (titer = 7.95% of active oxygen) | 1.26 | 200 | 67.7 |
| -Example 1 (titer = 7.42% of active oxygen) | 1.35 | 200 | 83.3 |
| -H 48 (titer = 5.5% of active oxygen) | 1.82 | 200 | 82.1 |
| -PBS (titer = 10% of active oxygen) + TAED | 1.0 + 0.8 | 200 | 79.7 |
| -PBS (titer = 10% of active oxygen) | 1.0 | 200 | 68.8 |

TABLE 2

| Tests carried out acid pH (2-3) | | | |
|---|---|---|---|
| Compound | Amounts used in the tests (grams) | Initial concentration of total active oxygen (mg/l) | % Bleaching |
| -Example 4 (titer = 5.93% of active oxygen) | 1.69 | 200 | 84.3 |
| -Example 7 (titer = 5.99% of active oxygen) | 1.67 | 200 | 82.8 |
| -Example 6 (titer = 4.87% of active oxygen) | 2.05 | 200 | 79.9 |
| -H 48 (titer = 5.5% of active oxygen) | 1.82 | 200 | 60.0 |

## Claims

**Claims for the following Contracting States : AT, BE, CH, DE, FR, GB, IT, LI, NL, SE**

**1.** Salts of amino-(poly)percarboxylic acids having the general formula (I):

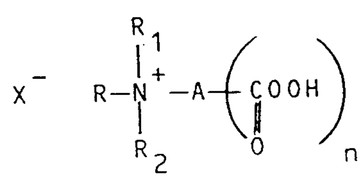

(I)

wherein

R, $R_1$ and $R_2$, which may be the same or different, represent hydrogen or optionally substituted alkyl or two of said groups R, $R_1$ and $R_2$ are combined to form an optionally substituted, aliphatic, heterocyclic ring with the nitrogen atom to which they are attached;

A is selected from optionally substituted alkylene, cycloalkylene, arylene, cycloalkylene-alkylene,

alkylene-cycloalkylene, arylene - alkylene or alkylene-arylene, said cycloalkylene or arylene units of the above groups being optionally fused with one or more cycloaliphatic groups and said alkylene units optionally having interspersed $CONR_3$ groups wherein $R_3$ is hydrogen, alkyl or aryl and said optional substituents of the above alkyl groups, heterocyclic rings and groups A being selected from halogen, OH, $NO_2$, lower alkoxy and carboxylic groups;
X represents $HSO_4$ or $CH_3SO_3$; and
n is an integer of from 1 to 6, preferably 1 or 2.

**2.** Salts according to claim 1, wherein R, $R_1$ and $R_2$ in formula (I) are selected from hydrogen and optionally substituted straight or branched $C_{1-5}$-alkyl groups.

**3.** Salts according to any one of claims 1 and 2, wherein two of the groups R, $R_1$ and $R_2$ are combined to result in a straight or branched, optionally substituted $C_{4-6}$ alkylene group, which, together with the nitrogen atom it is attached to, forms a heterocyclic ring.

**4.** Salts according to one or more of claims 1 to 3, wherein A represents linear or branched $C_{1-20}$-, preferably $C_{1-15}$-alkylene, $C_{3-12}$-cycloalkylene, $C_{6-14}$-arylene, $C_{3-12}$-cycloalkylene-$C_{1-5}$-alkylene, $C_{1-5}$-alkylene-$C_{3-12}$-cycloalkylene, $C_{6-14}$-arylene-$C_{1-5}$-alkylene or $C_{1-5}$-alkylene-$C_{6-14}$-arylene.

**5.** Salts according to claim 4, wherein the arylene unit of said arylene containing groups A is fused with one or more cycloaliphatic groups.

**6.** Salts according to one or more of the preceding claims, wherein at least one of said groups R, $R_1$ and $R_2$ is substituted by one or several, optionally different substituents selected from F, Cl, OH, $NO_2$, lower alkoxy and carboxylic groups.

**7.** Salts according to one or more of the preceding claims, wherein A represents alkylene or contains an alkylene unit, said alkylene chain optionally having interspersed $CONR_3$ groups wherein $R_3$ is hydrogen, lower alkyl or aryl.

**8.** Compounds according to claim 1, i.e. (percarboxymethyl)-trimethylammonium bisulfate, (2-percarboxyethyl)-ammonium methane sulfonate or bisulfate, (3-percarboxypropyl)-ammonium methane sulfonate or bisulfate, (4-percarboxybutyl)-ammonium methane sulfonate or bisulfate, (5-percarboxypentyl)-ammonium methane sulfonate or bisulfate, (10-percarboxydecyl)-ammonium methane sulfonate or bisulfate, (10-percarboxydecyl)-dimethylammonium bisulfate, (11-percarboxyundecyl)ammonium methane sulfonate or bisulfate, (11-percarboxyundecyl)-dimethylammonium methane sulfonate, the methane sulfonic acid salts of 3-piperidine-perpropionic acid, glycyl-amino-peracetic acid, 3-amino-perbenzoic acid, 5-amino-di-perisophthalic acid and 4-amino-phenyl-peracetic acid and the sulfuric acid salt of 2-amino-mono-persuccinic acid.

**9.** Process for preparing the salts according to any one of the preceding claims, characterized in that a substrate, i.e. an amino-(poly)-carboxylic acid or ester thereof corresponding to the desired salt of formula (I) or a quaternary salt of said acid or ester, is reacted with (concentrated) $H_2O_2$ in a medium selected from concentrated $H_2SO_4$ and $CH_3SO_3H$ and that the resulting salt of formula (I) is then separated from the reaction mixture by addition of tetrahydrofuran, ethyl acetate or a mixture thereof.

**10.** Process for preparing the salts according to claim 1, characterized in that a substrate corresponding to the desired salt of formula (I), i.e. an amino-(poly)carboxylic acid or ester thereof wherein at least one of R, $R_1$ and $R_2$ is hydrogen, or a quaternary salt of said acid or ester, is converted to the corresponding $HSO_4$- or $CH_3SO_3$-salt which salt is then reacted with (concentrated) $H_2O_2$ in a medium selected from concentrated $H_2SO_4$ and $CH_3SO_3H$ and that the salt of formula (I) thus obtained is separated from the reaction mixture by addition of tetrahydrofuran, ethyl acetate or a mixture thereof.

**11.** Process according to any one of claims 9 and 10, characterized in that the substrate is selected from 4-amino-butyric acid, 3-amino-propionic acid, (carboxymethyl)-trimethylammonium hydroxide or chloride, 3-piperidine-propionic acid, 11-amino-undecanoic acid, 12-amino-dodecanoic acid, glycyl-glycine, 3-amino-benzoic acid, 5-amino-isophthalic acid, 4-amino-phenyl-acetic acid, 5-amino-valeric acid, 6-amino-caproic acid, L-aspartic acid, N,N-dimethylamino lauric acid, N,N-dimethylamino undecanoic acid

or esters of said acids.

**12.** Process according to any one of claims 9 to 11, characterized in that the amino-(poly)carboxylic acid (ester) or ist quaternary salt is gradually reacted with $H_2O_2$ having a concentration of from about 70 to about 90% by weight in concentrated $H_2SO_4$ or $CH_3SO_3H$ at a temperature of from about 15 to about 50 °C.

**13.** Process according to any one of claims 9 to 12, characterized in that the amount of $H_2SO_4$ or $CH_3SO_3H$ is not less than 2 moles, preferably about 2 to 30 moles and particularly about 7 to 10 moles, per mole of substrate.

**14.** Process according to any one of claims 9 to 13, characterized in that the amount of $H_2O_2$ used is not less than 1 mole, preferably about 1 to 6 moles, and particularly 1.2 to about 2 moles, per mole of substrate.

**15.** Process according to any one of claims 9 to 14, characterized in that at the end of the reaction the molar ratio of $H_2SO_4$ or $CH_3SO_3H$ to the total $H_2O$ present is not lower than 1.5, preferably from about 1.5 to 10, and particularly from 4 to 6.

**16.** Process according to any one of claims 1 to 15, characterized in that the amount of THF and/or ethyl acetate used is not lower than 4 liters per mole of substrate.

**17.** Process according to any one of claims 1 to 16, characterized in that the THF and/or ethyl acetate is added at a temperature not higher than about 10 °C.

**18.** Use of the salts according to any one of claims 1 to 8 as bleaching agents in detergent formulations.

**Claims for the following Contracting State: ES**

**1.** Process for preparing salts of amino-(poly)percarboxylic acids having the general formula (I):

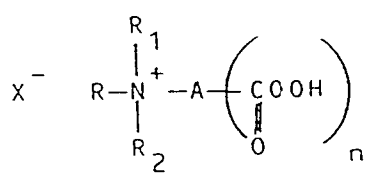

(I)

wherein
R, $R_1$ and $R_2$, which may be the same or different, represent hydrogen or optionally substituted alkyl or two of said groups R, $R_1$ and $R_2$ are combined to form an optionally substituted, aliphatic, heterocyclic ring with the nitrogen atom to which they are attached;
A is selected from optionally substituted alkylene, cycloalkylene, arylene, cycloalkylene-alkylene, alkylene-cycloalkylene, arylene - alkylene or alkylene-arylene, said cycloalkylene or arylene units of the above groups being optionally fused with one or more cycloaliphatic groups and said alkylene units optionally having interspersed $CONR_3$ groups wherein $R_3$ is hydrogen, alkyl or aryl and said optional substituents of the above alkyl groups, heterocyclic rings and groups A being selected from halogen, OH, $NO_2$, lower alkoxy and carboxylic groups;
X represents $HSO_4$ or $CH_3SO_3$; and
n is an integer of from 1 to 6, preferably 1 or 2,
characterized in that a substrate,
i.e. an amino-(poly)carboxylic acid or ester thereof corresponding to the desired salt of formula (I) or a quaternary salt of said acid or ester, is reacted with (concentrated) $H_2O_2$ in a medium selected from concentrated $H_2SO_4$ and $CH_3SO_3H$ and that the resulting salt of formula (I) is then separated from the reaction mixture by addition of tetrahydrofuran, ethyl acetate or a mixture thereof.

**2.** Process according to claim 1, wherein R, $R_1$ and $R_2$ in formula (I) are selected from hydrogen and optionally substituted straight or branched $C_{1-5}$-alkyl groups.

**3.** Process according to any one of claims 1 and 2, wherein two of the groups R, $R_1$ and $R_2$ are combined to result in a straight or branched, optionally substituted $C_{4-6}$ alkylene group, which, together with the nitrogen atom it is attached to, forms a heterocyclic ring.

**4.** Process according to one or more of claims 1 to 3, wherein A represents linear or branched $C_{1-20}$-, preferably $C_{1-15}$-alkylene, $C_{3-12}$-cycloalkylene, $C_{6-14}$-arylene, $C_{3-12}$-cycloalkylene-$C_{1-5}$-alkylene, $C_{1-5}$-alkylene-$C_{3-12}$-cycloalkylene, $C_{6-14}$-arylene-$C_{1-5}$-alkylene or $C_{1-5}$-alkylene-$C_{6-14}$-arylene.

**5.** Process according to claim 4, wherein the arylene unit of said arylene containing groups A is fused with one or more cycloaliphatic groups.

**6.** Process according to one or more of the preceding claims, wherein at least one of said groups R, $R_1$ and $R_2$ is substituted by one or several, optionally different substituents selected from F, Cl, OH, $NO_2$, lower alkoxy and carboxylic groups.

**7.** Process according to one or more of the preceding claims, wherein A represents alkylene or contains an alkylene unit, said alkylene chain optionally having interspersed $CONR_3$ groups wherein $R_3$ is hydrogen, lower alkyl or aryl.

**8.** Process according to claim 1, wherein the compounds of the general formula (I) are: (percarboxymethyl)trimethylammonium bisulfate, (2-percarboxyethyl)-ammonium methane sulfonate or bisulfate, (3-percarboxypropyl)-ammonium methane sulfonate or bisulfate, (4-percarboxybutyl)-ammonium methane sulfonate or bisulfate, (5-percarboxypentyl)-ammonium methane sulfonate or bisulfate, (10-percarboxydecyl)-ammonium methane sulfonate or bisulfate, (10-percarboxydecyl)-dimethylammonium bisulfate, (11-percarboxyundecyl)ammonium methane sulfonate or bisulfate, (11-percarboxyundecyl)-dimethylammonium methane sulfonate, the methane sulfonic acid salts of 3-piperidine-perpropionic acid, glycyl-amino-peracetic acid, 3-amino-perbenzoic acid, 5-amino-di-perisophthalic acid and 4-amino-phenyl-peracetic acid and the sulfuric acid salt of 2-amino-mono-persuccinic acid.

**9.** Process according to claim 1, characterized in that a substrate corresponding to the desired salt of formula (I), i.e. an amino-(poly)carboxylic acid or ester thereof wherein at least one of R, $R_1$ and $R_2$ is hydrogen, or a quaternary salt of said acid or ester, is converted to the corresponding $HSO_4$- or $CH_3SO_3$-salt which salt is then reacted with (concentrated) $H_2O_2$ in a medium selected from concentrated $H_2SO_4$ and $CH_3SO_3H$ and that the salt of formula (I) thus obtained is separated from the reaction mixture by addition of tetrahydrofuran, ethyl acetate or a mixture thereof.

**10.** Process according to any one of claims 1 and 9, characterized in that the substrate is selected from 4-amino-butyric acid, 3-amino-propionic acid, (carboxymethyl)-trimethylammonium hydroxide or chloride, 3-piperidine-propionic acid, 11-amino-undecanoic acid, 12-amino-dodecanoic acid, glycyl-glycine, 3-amino-benzoic acid, 5-amino-isophthalic acid, 4-amino-phenyl-acetic acid, 5-amino-valeric acid, 6-amino-caproic acid, L-aspartic acid, N,N-dimethylamino lauric acid, N,N-dimethylamino undecanoic acid or esters of said acids.

**11.** Process according to any one of claims 1 to 10, characterized in that the amino-(poly)carboxylic acid (ester) or its quaternary salt is gradually reacted with $H_2O_2$ having a concentration of from about 70 to about 90% by weight in concentrated $H_2SO_4$ or $CH_3SO_3H$ at a temperature of from about 15 to about 50 °C.

**12.** Process according to any one of claims 1 to 11, characterized in that the amount of $H_2SO_4$ or $CH_3SO_3H$ is not less than 2 moles, preferably about 2 to 30 moles and particularly about 7 to 10 moles, per mole of substrate.

**13.** Process according to any one of claims 1 to 12, characterized in that the amount of $H_2O_2$ used is not less than 1 mole, preferably about 1 to 6 moles, and particularly 1.2 to about 2 moles, per mole of substrate.

14. Process according to any one of claims 1 to 13, characterized in that at the end of the reaction the molar ratio of $H_2SO_4$ or $CH_3SO_3H$ to the total $H_2O$ present is not lower than 1.5, preferably from about 1.5 to 10, and particularly from 4 to 6.

15. Process according to any one of claims 1 to 14, characterized in that the amount of THF and/or ethyl acetate used is not lower than 4 liters per mole of substrate.

16. Process according to any one of claims 1 to 15, characterized in that the THF and/or ethyl acetate is added at a temperature not higher than about 10 °C.

**Patentansprüche**
**Patentansprüche für folgende Vertragsstaaten : AT, BE, CH, DE, FR, GB, IT, LI, NL, SE**

1. Salze von Amino-(poly)percarbonsäuren der allgemeinen Formel (I):

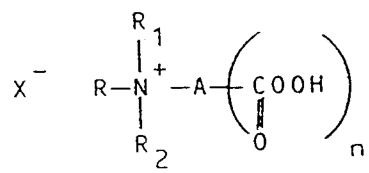

(I)

worin
R, $R_1$ und $R_2$, die gleich oder voneinander verschieden sein können, Wasserstoff oder fakultativ substituiertes Alkyl bedeuten oder zwei der Gruppen R, $R_1$ und $R_2$ kombiniert werden, um mit dem Stickstoffatom, an das sie gebunden sind, einen fakultativ substituierten aliphatischen heterocyclischen Ring zu bilden;
A ausgewählt ist aus fakultativ substituiertem Alkylen, Cycloalkylen, Arylen, Cycloalkylenalkylen, Alkylencycloalkylen, Arylenalkylen oder Alkylenarylen, wobei die Cycloalkylen- oder Aryleneinheiten der obigen Gruppen fakultativ mit einer oder mehreren cycloaliphatischen Gruppen kondensiert sind und die Alkyleneinheiten fakultativ eingeschobene $CONR_3$-Gruppen aufweisen, in welchen $R_3$ Wasserstoff, Alkyl oder Aryl ist, und die fakultativen Substituenten der obigen Alkylgruppen, heterocyclischen Ringe und Gruppen A aus Halogen, OH, $NO_2$, Niederalkoxy und Carboxylgruppen ausgewählt sind,
X $HSO_4$ oder $CH_3SO_3$ bedeutet und
n eine ganze Zahl von 1 bis 6, vorzugsweise 1 oder 2, ist.

2. Salze nach Anspruch 1, worin R, $R_1$ und $R_2$ in Formel (I) aus Wasserstoff und fakultativ substituierten geraden oder verzweigten $C_1$-$C_5$-Alkylgruppen ausgewählt sind.

3. Salze nach irgendeinem der Ansprüche 1 und 2, worin zwei der Gruppen R, $R_1$ und $R_2$ kombiniert werden, um eine gerade oder verzweigte, fakultativ substituierte $C_4$-$C_6$-Alkylengruppe zu ergeben, die zusammen mit dem Stickstoffatom, an das sie gebunden ist, einen heterocyclischen Ring bildet.

4. Salze nach einem oder mehreren der Ansprüche 1 bis 3, worin A lineares oder verzweigtes $C_1$-$C_{20}$-, vorzugsweise $C_1$-$C_{15}$-Alkylen, $C_3$-$C_{12}$-Cycloalkylen, $C_6$-$C_{14}$-Arylen, $C_3$-$C_{12}$-Cycloalkylen-$C_1$-$C_5$-alkylen, $C_1$-$C_5$-Alkylen-$C_3$-$C_{12}$-cycloalkylen, $C_6$-$C_{14}$-Arylen-$C_1$-$C_5$-alkylen oder $C_1$-$C_5$-Alkylen-$C_6$-$C_{14}$-arylen bedeutet.

5. Salze nach Anspruch 4, worin die Aryleneinheit der arylenhaltigen Gruppen A mit einer oder mehreren cycloaliphatischen Gruppen kondensiert ist.

6. Salze nach einem oder mehreren der vorhergehenden Ansprüche, worin mindestens eine der Gruppen R, $R_1$ und $R_2$ durch einen oder mehrere fakultativ verschiedene Substituenten, ausgewählt aus F, Cl, OH, $NO_2$, Niederalkoxy und Carboxylgruppen, substituiert ist.

7. Salze nach einem oder mehreren der vorhergehenden Ansprüche, worin A Alkylen bedeutet oder eine Alkyleneinheit enthält, wobei die Alkylenkette fakultativ eingeschobene $CONR_3$-Gruppen aufweist, in welchen $R_3$ Wasserstoff, Niederalkyl oder Aryl ist.

**8.** Verbindungen nach Anspruch 1, d.h. (Percarboxymethyl)-trimethylamoniumbisulfat, (2-Percarboxethyl)-ammoniummethansulfonat oder -bisulfat, (3-Percarboxrypropyl)-ammoniummethansulfonat oder -bisulfat, (4-Percarboxybutyl)-ammoniummethansulfonat oder -bisulfat, (5-Percarboxypentyl)-ammoniummethansulfonat oder -bisulfat, (10-Percarboxydecyl)-ammoniummethansulfonat oder -bisulfat, (10-Percarboxydecyl)-dimethylammoniumbisulfat, (11-Percarboxyundecyl)-ammoniummethansulfonat oder -bisulfat, (11-Percarboxyundecyl)-dimethylammoniummethansulfonat, die Methansulfonsäuresalze von 3-Piperidinperpropionsäure, Glycylaminoperessigsäure, 3-Aminoperbenzoesäure, 5-Aminodiperisophthalsäure, und 4-Aminophenylperessigsäure und das Schwefelsäuresalz von 2-Aminomonoperbernsteinsäure.

**9.** Verfahren zur Herstellung der Salze nach irgendeinem der vorhergehenden Ansprüche, dadurch gekennzeichnet, daß ein Substrat, d .h. eine Amino(poly)carbonsäure oder ein Ester derselben entsprechend dem gewünschten Salz der Formel (I) oder ein quaternäres Salz der Säure oder des Esters mit (konzentriertem) $H_2O_2$ in einem Medium, ausgewählt aus konzentrierter $H_2SO_4$ oder $CH_3SO_3H$, umgesetzt und das erhaltene Salz der Formel (I) dann von der Reaktionsmischung durch Zugabe von Tetrahydrofuran, Ethylacetat oder einer Mischung derselben abgetrennt wird.

**10.** Verfahren zur Herstellung der Salze nach Anspruch 1, dadurch gekennzeichnet, daß ein Substrat entsprechend dem gewühschten Salz der Formel (I), d.h. eine Amino-(poly)carbonsäure oder ein Ester derselben, worin mindestens eine der Gruppen R, $R_1$ und $R_2$ Wasserstoff ist, oder ein quaternäres Salz dieser Säure oder des Esters in das entsprechende $HSO_4$- oder $CH_3SO_3$-Salz umgewandelt wird, das dann mit (konzentriertem) $H_2O_2$ in einem Medium, ausgewählt aus konzentrierter $H_2SO_4$ und $CH_3SO_3H$, umgesetzt wird und daß das so erhaltene Salz der Formel (I) von der Reaktionsmischung durch Zugabe von Tetrahydrofuran, Ethylacetat oder einer Mischung derselben abgetrennt wird.

**11.** Verfähren nach irgendeinem der Ansprüche 9 und 10, dadurch gekennzeichnet, daß das Substrat ausgewählt ist aus: 4-Aminobuttersäure, 3-Aminopropionsäure, (Carboxymethyl)-trimethylammoniumhydroxid oder -chlorid, 3-Piperidinpropionsäure, 11-Aminoundecansäure, 12-Aminododecansäure, Glycylglyin, 3-Aminobenzoesäure, 5-Aminoisophthalsäure, 4- Aminophenylessigsäure, 5-Aminovaleriansäure, 6-Aminocapronsäure, L-Asparaginsäure, N,N-Dimethylaminolaurinsäure, N,N-Dimethylaminoundecansäure oder Estern dieser Säuren.

**12.** Verfahren nach irgendeinem der Ansprüche 9 bis 11, dadurch gekennzeichnet, daß die (der) Amino-(poly)carbonsäure(ester) oder deren quaternäres Salz allmählich mit $H_2O_2$ einer Konzentration von etwa 70 bis etwa 90 Gew.-% in konzentrierter $H_2SO_4$ oder $CH_3SO_3H$ bei einer Temperatur von etwa 15 bis etwa 50 °C umgesetzt wird.

**13.** Verfahren nach irgendeinem der Ansprüche 9 bis 12, dadurch gekennzeichnet, daß die Menge an $H_2SO_4$ oder $CH_3SO_3H$ nicht weniger als 2 Mol, vorzugsweise etwa 2 bis 30 Mol und insbesondere etwa 7 bis 10 Mol, pro Mol Substrat beträgt.

**14.** Verfahren nach irgendeinem der Ansprüche 9 bis 13, dadurch gekennzeichnet, daß die verwendete Menge $H_2O_2$ nicht weniger als 1 Mol, vorzugsweise etwa 1 bis 6 Mol und insbesondere 1,2 bis etwa 2 Mol, pro Mol Substrat beträgt.

**15.** Verfahren nach irgendeinem der Ansprüche 9 bis 14, dadurch gekennzeichnet, daß am Ende der Reaktion das molare Verhältnis von $H_2SO_4$ oder $CH_3SO_3H$ zum insgesamt vorliegenden $H_2O$ nicht unter 1,5, vorzugsweise von etwa 1,5 bis 10 und insbesondere von 4 bis 6, beträgt.

**16.** Verfahren nach irgendeinem der Ansprüche 1 bis 15, dadurch gekennzeichnet, daß die verwendete Menge an THF und/oder Ethylacetat nicht weniger als 4 l pro Mol Substrat beträgt.

**17.** Verfahren nach irgendeinem der Ansprüche 1 bis 16, dadurch gekennzeichnet, daß das THF und/oder Ethylacetat bei einer Temperatur nicht über etwa 10 °C zugefügt wird.

**18.** Verwendung der Salze nach irgendeinem der Ansprüche 1 bis 8 als Bleichmittel in Reinigungsmittelformulierungen.

**Patentansprüche für folgenden Vertragsstaat : ES**

**1.** Verfahren zur Herstellung von Salzen von Amino(poly)percarbonsäuren der allgemeinen Formel (I):

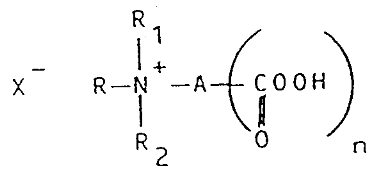

(I)

worin
R, $R_1$ und $R_2$, die gleich oder voneinander verschieden sein können, Wasserstoff oder fakultativ substituiertes Alkyl bedeuten oder zwei der Gruppen R, $R_1$ und $R_2$ kombiniert werden, um mit dem Stickstoffatom, an das sie gebunden sind, einen fakultativ substituierten aliphatischen heterocyclischen Ring zu bilden;
A ausgewählt ist aus fakultativ substituiertem Alkylen, Cycloalkylen, Arylen, Cycloalkylenalkylen, Alkylencycloalkylen, Arylenalkylen oder Alkylenarylen, wobei die Cycloalkylen- oder Aryleneinheiten der obigen Gruppen fakultativ mit einer oder mehreren cycloaliphatischen Gruppen kondensiert sind und die Alkyleneinheiten fakultativ eingeschobene $CONR_3$-Gruppen aufweisen, in welchen $R_3$ Wasserstoff, Alkyl oder Aryl ist, und die fakultativen Substituenten der obigen Alkylgruppen, heterocyclischen Ringe und Gruppen A aus Halogen, OH, $NO_2$, Niederalkoxy und Carboxylgruppen ausgewählt sind,
X $HSO_4$ oder $CH_3SO_3$ bedeutet und
n eine ganze Zahl von 1 bis 6, vorzugsweise 1 oder 2, ist,
dadurch gekennzeichnet, daß ein Substrat, d.h. eine Amino(poly)carbonsäure oder ein Ester derselben entsprechend dem gewünschten Salz der Formel (I) oder ein quaternärez Salz der Säure oder des Esters mit (konzentriertem) $H_2O_2$ in einem Medium, ausgewählt aus konzentrierter $H_2SO_4$ oder $CH_3SO_3H$, umgesetzt und das erhaltene Salz der Formel (I) dann von der Reaktionsmischung durch Zugabe von Tetrahydrofuran, Ethylacetat oder einer Mischung derselben abgetrennt wird.

**2.** Verfahren nach Anspruch 1, worin R, $R_1$ und $R_2$ in Formel (I) aus Wasserstoff und fakultativ substituierten geraden oder verzweigten $C_1$-$C_5$-Alkylgruppen ausgewählt sind.

**3.** Verfahren nach irgendeinem der Ansprüche 1 und 2, worin zwei der Gruppe R, $R_1$ und $R_2$ kombiniert werden, um eine gerade oder verzweigte, fakultativ substituierte $C_4$-$C_6$-Alkylengruppe zu ergeben, die zusammen mit dem Stickstoffatom, an das sie gebunden ist, einen heterocyclischen Ring bildet.

**4.** Verfahren nach einem oder mehreren der Ansprüche 1 bis 3, worin A lineares oder verzweigtes $C_1$-$C_{20}$-, vorzugsweise $C_1$-$C_{15}$-Alkylen, $C_3$-$C_{12}$-Cycloalkylen, $C_6$-$C_{14}$-Arylen, $C_3$-$C_{12}$-Cycloalkylen-$C_1$-$C_5$-alkylen, $C_1$-$C_5$-Alkylen-$C_3$-$C_{12}$-cycloalkylen, $C_6$-$C_{14}$-Arylen-$C_1$-$C_5$-alkylen oder $C_1$-$C_5$-Alkylen-$C_6$-$C_{14}$-arylen bedeutet.

**5.** Verfahren nach Anspruch 4, worin die Aryleneinheit der arylenhaltigen Gruppen A mit einer oder mehreren cycloaliphatischen Gruppen kondensiert ist.

**6.** Verfahren nach einem oder mehreren der vorhergehenden Ansprüche, worin mindestens eine der Gruppen R, $R_1$ und $R_2$ durch einen oder mehrere falkultativ verschiedene Substituenten, ausgewählt aus F, Cl, OH, $NO_2$, Niederalkoxy und Carboxylgruppen, substituiert ist.

**7.** Verfahren nach einem oder mehreren der vorhergehenden Ansprüche, worin A Alkylen bedeutet oder eine Alkyleneinheit enthält, wobei die Alkylenkette fakultativ eingeschobene $CONR_3$-Gruppen aufweist, in welchen $R_3$ Wasserstoff, Niederalkyl oder Aryl ist.

**8.** Verfahren nach Anspruch 1, worin die Verbindungen der allgemeinen Formel (I) (Percarboxymethyl)-trimethylamoniumbisulfat, (2-Percarboxyethyl) -ammoniummethansulfonat oder -bisulfat, (3-Percarboxypropyl)-ammoniumethansulfonat oder -bisulfat, (4-Percarboxybutyl)-ammoniunmethansulfonat oder -bisulfat, (5-Percarboxypentyl)-ammoniummethansulfonat oder -bisulfat, (10-Percarboxydecyl)-ammoniummethansulfonat oder -bisulfat, (10-Percarboxydecyl)-dimethylammoniumbisulfat, (11-Percarboxyundecyl)-ammoniummethansulfonat oder -bisulfat, (11-Percarboxyundecyl)-dimethylammoniummethansulfonat, die Methansulfonsäuresalze von 3-Piperidinperpropionsäure, Glycylaminoperessigsäure, 3-Aminoperbenzoesäure, 5-Aminodiperisophtbalsäure, und 4-Aminophenylperessigsäure und das Schwefelsäuresalz von 2-Aminomonoperbernsteinsäure sind.

**9.** Verfahren nach Anspruch 1, dadurch gekennzeichnet, daß ein Substrat entsprechend dem gewünschten Salz der Formel (I), d.h. eine Amino-(poly)carbonsäure oder ein Ester derselben, worin mindestens eine der Gruppen R, $R_1$ und $R_2$ Wasserstoff ist, oder ein quaternäres Salz dieser Säure oder des Esters in das entsprechende $HSO_4$- oder $CH_3SO_3$-Salz umgewandelt wird, das dann mit (konzentriertem) $H_2O_2$ in einem Medium, ausgewählt aus konzentrierter $H_2SO_4$ und $CH_3SO_3H$, umgesetzt wird und daß das so erhaltene Salz der Formel (I) von der Reaktionsmischung durch Zugabe von Tetrahydrofuran, Ethylacetat oder einer Mischung derselben abgetrennt wird.

**10.** Verfahren nach irgendeinem der Ansprüche 1 und 9 dadurch gekennzeichnet, daß das Substrat ausgewählt ist aus: 4-Aminobuttersäure, 3-Aminopropionsäure, (Carboxymethyl)-trimethylammoniumhydroxid oder -chlorid, 3-Piperidinpropionsäure, 11-Aminoundecansäure, 12-Aminododecansäure, Glycylglyin, 3-Aminobenzoesäure, 5-Aminoisophtbalsäure, 4-Aminophenylessigsäure, 5-Aminovaleriansäure, 6-Aminocapronsäure, L-Asparaginsäure, N,N-Dimethylaminolaurinsäure, N,N-Dimethylaminoundecansäure oder Estern dieser Säuren.

**11.** Verfahren nach irgendeinem der Ansprüche 1 bis 10, dadurch gekennzeichnet, daß die (der) Amino-(poly)carbonsäure(ester) oder deren quaternäres Salz allmählich mit $H_2O_2$ einer Konzentration von etwa 70 bis etwa 90 Gew.-% in konzentrierter $H_2SO_4$ oder $CH_3SO_3H$ bei einer Temperatur von etwa 15 bis etwa 50 °C umgesetzt wird.

**12.** Verfahren nach irgendeinem der Ansprüche 1 bis 11, dadurch gekennzeichnet, daß die Menge an $H_2SO_4$ oder $CH_3SO_3H$ nicht weniger als 2 Mol, vorzugsweise etwa 2 bis 30 Mol und insbesondere etwa 7 bis 10 Mol, pro Mol Substrat beträgt.

**13.** Verfahren nach irgendeinem der Ansprüche 1 bis 12, dadurch gekennzeichnet, daß die verwendete Menge $H_2O_2$ nicht weniger als 1 Mol, vorzugsweise etwa 1 bis 6 Mol und insbesondere 1,2 bis etwa 2 Mol, pro Mol Substrat beträgt.

**14.** Verfahren nach irgendeinem der Ansprüche 1 bis 13, dadurch gekennzeichnet, daß am Ende der Reaktion das klare Verhältnis von $H_2SO_4$ oder $CH_3SO_3H$ zum insgesamt vorliegenden $H_2O$ nicht unter 1,5, vorzugsweise von etwa 1,5 bis 10 und insbesondere von 4 bis 6, beträgt.

**15.** Verfahren nach irgendeinem der Ansprüche 1 bis 14, dadurch gekennzeichnet, daß die verwendete Menge an THF und/oder Ethylacetat nicht weniger als 4 l pro Mol Substrat beträgt.

**16.** Verfahren nach irgendeinem der Ansprüche 1 bis 15, dadurch gekennzeichnet, daß das THF und/oder Ethylacetat bei einer Temperatur nicht über etwa 10 °C zugefügt wird.

**Revendications**

**Revendications pour les Etats contractants suivants : AT, BE, CH, DE, FR, GB, IT, LI, NL, SE**

**1.** Sels d'acides amino-(poly)percarboxyliques de formule générale (I):

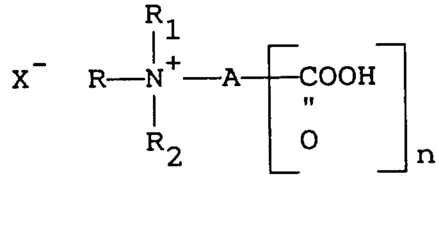

(I)

dans laquelle:

R, $R_1$ et $R_2$, qui peuvent être identiques ou différents, représentent un atome d'hydrogène ou un radical alkyle éventuellement substitué ou deux de ces dits groupes,

R, $R_1$, et $R_2$ sont combinés pour former un cycle hétérocyclique, aliphatique, éventuellement substitué avec l'atome d'azote auquel ils sont attachés;

A est sélectionné parmi les radicaux alkylène, cycloalkylène, arylène, cycloalkylène-alkylène, alkylènecycloalkylène, arylène-alkylène ou alkylène-arylène, éventuellement substitués, lesdites unités cycloalkylène ou arylène des groupes ci-dessus étant éventuellement fusionnés avec un ou plusieurs groupes cycloaliphatiques et lesdites unités alkylènes contenant éventuellement des groupes $CONR_3$ dispersés dans lesquels $R_3$ représente un atome d'hydrogène, un radical alkyle ou aryle, et lesdits substituants éventuels desdits radicaux alkyle, cycles hétérocycliques et groupe A étant choisis parmi un atome d'halogène, un groupe OH, $NO_2$, des radicaux carboxylique et alkoxy inférieurs;

X représente $HSO_4$ ou $CH_3SO_3$; et

n est un nombre entier compris entre 1 et 6, de préférence égal ä 1 ou 2.

**2.** Sels selon la revendication 1, dans lesquels R, $R_1$ et $R_2$ de la formule (I) sont sélectionnés parmi l'atome d'hydrogène et les groupes alkyles en $C_{1-5}$ linéaires ou ramifiés, éventuellément substitués.

**3.** Sels selon l'une quelconque des revendications 1 et 2, dans lesquels deux groupes R, $R_1$ et $R_2$ sont combinés pour donner un groupe alkylène en $C_4$-$C_6$, linéaire ou ramifié, éventuellement substitué, qui, avec l'atome d'azote auquel ils sont attachés forment un cycle hétérocyclique.

**4.** Sels selon l'une ou plusieurs des revendications 1 à 3, dans lesquels A représente un radical alkylène en $C_1$-$C_{20}$,de préférence $C_1$-$c_{15}$, cycloalkylène en $C_3$-$C_{12}$, arylène en $C_6$-$C_{14}$, cycloalkylène en $C_3$-$C_{12}$-alkylène en $C_1$-$C_5$, alkylène en $C_1$-$C_5$-cycloalkylène en $C_3$-$C_{12}$, arylène en $C_6$-$C_{14}$-alkylène en $C_1$-$C_5$ ou alkylène en $C_1$-$C_5$-arylène en $C_6$-$C_{14}$ linéaires ou ramifiés.

**5.** Sels selon la revendication 4, dans lesquels l'unité arylène dudit groupe A contenant l'arylène fusionne avec un ou plusieurs groupes cycloaliphatiques.

**6.** Sels selon l'une ou plusieurs des revendications précédentes, dans lesquelles au moins un desdits groupes R, $R_1$ et $R_2$ sont substitués par un ou plusieurs substituants éventuellement différents sélectionnés parmi F, Cl, OH, $NO_2$, des groupes alcoxy inférieurs et des groupes carboxyliques.

**7.** Sels selon l'une ou plusieurs des revendications précédentes, dans lesquels A représente l'unité alkylène ou contient une unité alkylène, ladite chaîne alkylène contenant éventuellement des groupes $CONR_3$ dispersés, dans lesquels $R_3$ représente un atome d'hydrogène, un radical alkyle ou aryle inférieur.

**8.** Composé selon la revendication 1, c'est-à-dire: bisulfate de (percarboxyméthyl)-triméthylammonium, bisulfate ou sulfonate de méthane de (2-percarboxyéthyl-ammonium, bisulfate ou sulfonate de méthane de (3-percarboxypropyl)-ammonium, bisulfate ou sulfonate de méthane de (4-percarboxybutyl)-ammonium, bisulfate ou sulfonate de méthane de (5-percarboxypentyl)-ammonium, méthane sulfonate ou bisulfate de (10-percarboxydécyl)-ammonium, bisulfate de (10-percarboxydécyl)-diméthylammonium, bisulfate ou sulfonate de méthane de (11-percarboxyundécyl)ammonium, méthane sulfonate de (11-percarboxyundécyl)-diméthylammonium, les sels d'acide méthanesulfonique d'acide de 3-pipéridine-perpropionique, acide glycyl-amino-peracétique, acide 3-amino-perbenzoïque, acide 5-amino-di-perisophtalique et acide 4-amino-phényl-peracétique et sel d'acide sulfurique de l'acide 2-amino-monopersuccinique.

**9.** Procédé pour la préparation de sels selon l'une quelconque des revendications précédentes, caractérisé en ce que le substrat, c'est-à-dire un acide amino-(poly)carboxylique ou un ester correspondant au sel désiré de formule (I) ou un sel quaternaire de ce dit acide ou ester, réagit avec $H_2O_2$ (concentré) dans un milieu sélectionné parmi $H_2SO_4$ et $CH_3SO_3H$ concentrés et en ce que le sel résultant de formule (I) est alors séparé du milieu réactionnel par addition de tétrahydrofuranne, acétate d'éthyle ou un mélange de ces composés.

**10.** Procédé de préparation de sels selon la revendication 1, caractérisé en ce que le substrat correspondant au sel désiré de formule (I), c'est-à-dire un acide amino-(poly)carboxylique ou un ester dans

lequel au moins un des groupes R, $R_1$ et $R_2$ représente un atome d'hydrogène ou un sel quaternaire de ce dit acide ou ester est converti en un sel correspondant $HSO_4$- ou $CH_3SO_3$- lequel réagit alors avec $H_2O_2$ (concentré) dans un milieu sélectionné parmi $H_2SO_4$ et $CH_3SO_3H$ concentré et en ce que le sel de formule (I) ainsi obtenu est séparé du mélange réactionnel par addition de tétrahydrofuranne, acétate d'éthyle ou d'un mélange de ces composés.

**11.** Procédé selon l'une quelconque des revendications 9 et 10, caractérisé en ce que le substrat est sélectionné parmi l'acide 4-amino-butyrique, l'acide 3-amino-propionique, l'hydroxyde ou le chlorure de (carboxyméthyl)-triméthylammonium, l'acide 3-pipéridine-propionique, l'acide 11-aminoundécanoïque, l'acide 12-amino-dodécanoïque, la glycylglycine, l'acide 3-amino-benzoïque, l'acide 5-amino-isophtalique, l'acide 4-amino-phénylacétique, l'acide 5-aminovalérique, l'acide 6-amino-caproïque, l'acide L-aspartique, l'acide N,N- diméthylamino-laurique, l'acide N,N-diméthylamino-undécanoïque; ou les esters de ces dits acides.

**12.** Procédé selon l'une quelconque des revendications 9 à 11, caractérisé en ce que l'acide (ou ester) amino-(poly)-carboxylique ou son sel quaternaire réagit graduellement avec $H_2O_2$ ayant une concentration d'environ 70 à environ 90% en poids dans $H_2SO_4$ ou $CH_3SO_3H$ concentrés à une température comprise entre 15 et environ 50 °C.

**13.** Procédé selon l'une quelconque des revendications 9 à 12, caractérisé en ce que la quantité d'$H_2SO_4$ ou $CH_3SO_3H$ n'est pas inférieure à 2 moles, de préférence comprise entre 2 et 30 moles et plus particulièrement entre 7 et 10 moles, par mole de substrat.

**14.** Procédé selon l'une quelconque des revendications 9 à 13, caractérisé en ce que la quantité d'$H_2O_2$ utilisée n'est pas inférieure à 1 mole, de préférence entre 1 et 6 moles, et plus particulièrement entre 1,2 et environ 2 moles, par mole de substrat.

**15.** Procédé selon l'une quelconque des revendications 9 à 14, caractérisé en ce qu'à la fin de la réaction le rapport molaire $H_2SO_4$ ou $CH_3SO_3H/H_2O$ total présent n'est pas inférieur à 1,5, de préférence entre environ 1,5 et 10, et plus particulièrement entre 4 et 6.

**16.** Procédé selon l'une quelconque des revendications 1 à 15, caractérisé en ce que la quantité de THF et/ou acétate d'éthyle utilisée n'est pas inférieure à 4 litres par mole de substrat.

**17.** Procédé selon l'une quelconque des revendications 1 à 16, caractérisé en ce que le THF et/ou l'acétate d'éthyle est ajouté à une température qui n'est pas supérieure à environ 10 °C.

**18.** L'utilisation des sels selon l'une quelconque des revendications 1 à 8 comme agents de blanchiment dans des formulations détergentes.

**Revendications pour l'Etat contractant suivant : ES**

**1.** Procédé de préparation des sels d'acides amino-(poly)percarboxyliques de formule générale (I):

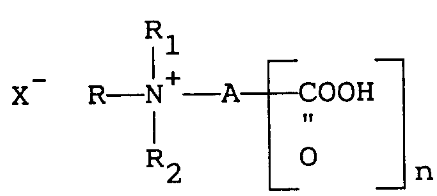

(I)

dans laquelle:

R, $R_1$ et $R_2$, qui peuvent être identiques ou différents, représentent un atome d'hydrogène ou un radical alkyle éventuellement substitué, ou deux de ces dits groupes R, $R_1$, et $R_2$ sont combinés pour former un cycle hétérocyclique, aliphatique, éventuellement substitué avec l'atome d'azote auquel ils sont attachés;

A est sélectionné parmi les radicaux alkylène, cycloalkylène, arylène, cycloalkylène-alkylène, alkylène-cycloalkylène, arylène-alkylène ou alkylène-arylène, éventuellement substitués, lesdites unités cycloalkylène ou arylène des groupes ci-dessus étant éventuellement fusionnées avec un ou plusieurs groupes cycloaliphatiques et lesdites unités alkylènes contenant éventuellement des groupes $CONR_3$ dispersés dans lesquels $R_3$ représente un atome d'hydrogène, un radical alkyle ou aryle, et lesdits substituants éventuels desdits radicaux alkyle, cycles hétérocycliques et groupe A étant choisis parmi un atome d'halogène, un groupe OH, $NO_2$, des radicaux carboxylique et alkoxy inférieurs;

X représente $HSO_4$ ou $CH_3SO_3$; et

n est un nombre entier compris entre 1 et 6, de préférence égal à 1 ou 2,

caractérisé en ce qu'un substrat, c'est-à-dire un acide amino-(poly)carboxylique ou un ester correspondant au sel désiré de formule (I) ou un sel quaternaire de ce dit acide ou ester, réagit avec $H_2O_2$ - (concentré) dans un milieu sélectionné parmi $H_2SO_4$ et $CH_3SO_3H$ concentrés et en ce que le sel résultant de formule (I) est alors séparé du milieu réactionnel par addition de tétrahydrofuranne, acétate d'éthyle ou un mélange de ces composés.

**2.** Un procédé selon la revendication 1, caractérisé en ce que R, $R_1$ et $R_2$ de la formule (I) sont sélectionnés parmi l'atome d'hydrogène et les groupes alkyles en $C_{1-5}$ linéaires ou ramifiés, éventuellement substitués.

**3.** Un procédé selon l'une quelconque des revendications 1 et 2, caractérisé en ce que deux groupes R, $R_1$ et $R_2$ sont combinés pour donner un groupe alkylène en $C_4$-$C_6$, linéaire ou ramifié, éventuellement substitué, qui, avec l'atome d'azote auquel ils sont attachés forment un cycle hétérocyclique.

**4.** Un procédé selon l'une ou plusieurs des revendications 1 à 3, caractérisé en ce que A représente un radical alkylène en $C_1$-$C_{20}$, de préférence $C_1$-$C_{15}$, cycloalkylène en $C_3$-$C_{12}$, arylène en $C_6$-$C_{14}$, cycloalkylène en $C_3$-$C_{12}$-alkylène en $C_1$-$C_5$, alkylène en $C_1$-$C_5$-cycloalkylène en $C_3$-$C_{12}$, arylène en $C_6$-$C_{14}$-alkylène en $C_1$-$C_5$ ou alkylène en $C_1$-$C_5$-arylène en $C_6$-$C_{14}$ linéaires ou ramifiés.

**5.** Un procédé selon la revendication 4, caractérisé en ce que l'unité arylène dudit groupe A contenant l'arylène fusionne avec un ou plusieurs groupes cycloaliphatiques.

**6.** Un procédé selon l'une ou plusieurs des revendications précédentes, caractérisé en ce qu' au moins un desdits groupes R, $R_1$ et $R_2$ est substitué par un ou plusieurs substituants éventuellement différents sélectionnés parmi F, Cl, OH, $NO_2$, les groupes alcoxy inférieurs et les groupes carboxyliques.

**7.** Un procédé selon l'une ou plusieurs des revendications précédentes, caractérisé en ce que A représente l'unité alkylène ou contient une unité alkylène, ladite chaîne alkylène contenant éventuellement des groupes $CONR_3$ dispersés, dans lesquels $R_3$ représente un atome d'hydrogène, un radical alkyle ou aryle inférieur.

**8.** Un procédé selon la revendication 1, caractérisé en ce que les dérivés représentés par la formule générale I sont: bisulfate de (percarboxyméthyl)-triméthylammonium, bisulfate ou sulfonate de méthane de (2-percarboxyéthyl)-ammonium, bisulfate ou sulfonate de méthane de (3-percarboxypropyl)-ammonium, bisulfate ou sulfonate de méthane de (4-percarboxybutyl)-ammonium, bisulfate ou sulfonate de méthane de (5-percarboxypentyl)-ammonium, méthane sulfonate ou bisulfate de (10-percarboxydécyl)-ammonium, bisulfate de (10-percarboxydécyl)-diméthylammonium, bisulfate ou sulfonate de méthane de (11-percarboxyundécyl)ammonium méthane sulfonate de (11-percarboxyundécyl)-diméthylammonium, les sels d'acide méthanesulfonique d'acide de 3-pipéridine-perpropionique, acide glycyl-aminoperacétique, acide 3-amino-phényl-per-acide 5-amino-di-perisophtalique et acide 4-amino-phényl-peracétique et sel d'acide sulfurique de l'acide 2-amino-monopersuccinique.

**9.** Un procédé selon la revendication 1, caractérisé en ce qu'un substrat correspondant au sel désiré de formule (I), c'est-à-dire un acide amino-(poly)carboxylique ou un ester dans lequel au moins un des groupes R, $R_1$ et $R_2$ représente un atome d'hydrogène ou un sel quaternaire de ce dit acide ou ester est converti en un sel correspondant $HSO_4$- ou $CH_3SO_3$-, lequel réagit alors avec $H_2O_2$ (concentré) dans un milieu sélectionné parmi $H_2SO_4$ et $CH_3SO_3H$ concentré et en ce que le sel de formule (I) ainsi obtenu est séparé du mélange réactionnel par addition de tétrahydrofuranne, acétate d'éthyle ou d'un

mélange de ces composes.

**10.** Un procédé selon l'une quelconque des revendications 1 et 9, caractérisé en ce que le substrat est sélectionné parmi l'acide 4-amino-butyrique, l'acide 3-amino-propionique, l'hydroxyde ou le chlorure de (carboxyméthyl)-triméthylammonium, l'acide 3-pipéridine-propionique, l'acide 11-aminoundécanoïque, l'acide 12-amino-dodécanoïque, la glycylglycine, l'acide 3-amino-benzoïque, l'acide 5-amino-isophtalique, l'acide 4-amino-phénylacétique, l'acide 5-amino-valérique, l'acide 6-amino-caproïque, l'acide L-aspartique, l'acide N,N- diméthylamino-laurique, l'acide N,N-diméthylamino-undécanoïque; ou les esters de ces dits acides.

**11.** Un procédé selon l'une quelconque des revendications 1 à 10, caractérisé en ce que l'acide (ou ester) amino-(poly)carboxylique ou son sel quaternaire réagit graduellement avec $H_2O_2$ ayant une concentration d'environ 70 à environ 90% en poids dans $H_2SO_4$ ou $CH_3SO_3H$ concentrés à une température comprise entre 15 et environ 50 °C.

**12.** Un procédé selon l'une quelconque des revendications 1 à 11, caractérisé en ce que la quantité d'$H_2SO_4$ ou $CH_3SO_3H$ n'est pas inférieure à 2 moles, de préférence comprise entre 2 et 30 moles et plus particulièrement entre 7 et 10 moles, par mole de substrat.

**13.** Un procédé selon l'une quelconque des revendications 1 à 12, caractérisé en ce que la quantité d'$H_2O_2$ utilisée n'est pas inférieure à 1 mole, de préférence entre 1 et 6 moles, et plus particulièrement entre 1,2 et environ 2 moles, par mole de substrat.

**14.** Un procédé selon l'une quelconque des revendications 1 à 13, caractérisé en ce qu'à la fin de la réaction le rapport molaire $H_2SO_4$ ou $CH_3SO_3H/H_2O$ total présent n'est pas inférieur à 1,5, de préférence entre environ 1,5 et 10, et plus particulièrement entre 4 et 6.

**15.** Un procédé selon l'une quelconque des revendications 1 à 14, caractérisé en ce que la quantité de THF et/ou acétate d'éthyle utilisée n'est pas inférieure à 4 litres par mole de substrat.

**16.** Un procédé selon l'une quelconque des revendications 1 à 15, caractérisé en ce que le THF et/ou l'acétate d'éthyle est ajouté à une température qui n'est pas supérieure à environ 10 °C.